# EUROPEAN PATENT APPLICATION

(11) **EP 4 454 548 A1**
(43) Date of publication of application: **30.10.2024**
(21) Application number: 23169699.8
(22) Date of filing: 25.04.2023
(51) Int. Cl.: A61B 5/0235, A61B 5/00, A61B 5/022

(54) **AIR FLOW CONTROL FOR HEMODYNAMIC MEASUREMENT CUFF**

(71) Applicant: Koninklijke Philips N.V., 5656 AG Eindhoven (NL)
(72) Inventor: HILGERS, Achim Rudolf, 5656 AG Eindhoven (NL); PELSSERS, Eduard Gerard Marie, 5656 AG Eindhoven (NL); DAVIDOIU, Valentina-Geta, 5656 AG Eindhoven (NL); MENA BENITO, Maria Estrella, 5656 AG Eindhoven (NL)
(74) Representative: Philips Intellectual Property & Standards

(57) **Abstract**

A non-invasive hemodynamic monitoring system, wherein a pressure or flow modulator is incorporated in-line along at least a portion of a fluid supply path to a measurement cuff, and wherein the system includes static or active means for permitting a user to selectively operate the system in a first mode in which the pressure modulator is active and a second mode in which the pressure modulator is not active, and optionally a third mode in which the pressure modulator is partially active.

## Description

### FIELD OF THE INVENTION

The present invention relates to the field of hemodynamic measurement using an inflatable cuff, and in particular to the air flow control in such a context.

### BACKGROUND OF THE INVENTION

Hemodynamic monitoring describes the dynamics of blood flow in a vascular system and plays an important role in monitoring critically ill patients. Usually, hemodynamic monitoring is based on complex invasive measurements of the arterial blood pressure as well as cardiac output/parameter.

Hemodynamic monitoring refers to the measurement and analysis of blood flow characteristics in a vascular system of a patient. It is particularly important in the context of monitoring of critically ill patients as changes in blood flow and blood pressure can have a significant impact on health status and are indicative of various other physiological conditions of the patient. Hemodynamic monitoring in the critical context typically employs use of invasive measurement methods because these provide the most accurate measurements. Invasive methods involve for example arterial catheterization or pulmonary artery catheterization.

It would be of value in the medical field to provide non-invasive hemodynamic monitoring methods that are capable of rivalling the accuracy and reliability of non-invasive methods.

The most common non-invasive method of monitoring blood pressure and other hemodynamic measurements is through the use of an inflatable cuff attached to the arm of the patient. Such systems are known as sphygmomanometers.

State of the art sphygmomanometers are unable to provide continuous hemodynamic monitoring because the principle of operation involves cycling through a sequence of applied pressure states of the cuff to obtain a single output blood pressure measurement.

### SUMMARY OF THE INVENTION

In seeking to develop improvements in the area of cuff-based hemodynamic monitoring, the inventors associated with the present application have identified that a bottleneck in development is the lack of configurability in the air flow characteristics provided by the air flow source. For patients of different body mass (e.g. adults versus children), it has been found that it may be beneficial to be able to vary the pressure level of the air supplied to the cuff depending upon the patient size and age.

Embodiments of the present invention provide a particularly simple and efficient way of providing configurability of air flow characteristics of the pressurized air source to the cuff.

The invention is defined by the independent claims. The dependent claims define advantageous embodiments.

According to examples in accordance with an aspect of the invention, there is provided a system comprising: a cuff with an inflatable bladder for adjusting a pressure applied by the cuff to a body part of a patient around which the cuff is worn; a source of pressurized air for use in inflating the bladder of the cuff; and an air flow path for fluidly connecting the source of pressurized air and the inflatable bladder of the cuff. The system further comprises an air pressure modulation means for disposal along the air flow path between the source of pressurized air and the inflatable bladder of the cuff and adapted to provide a modulation/modification of a pressure of air arriving at the inflatable bladder of the cuff. Advantageously, the system is selectively configurable in each of at least two modes: a first mode in which the air pressure modulation means is arranged so as to provide the air pressure modulation/modification, and a second mode in which the air pressure modulation means is arranged so as to not provide the air pressure modulation/modification.

By way of example, in the first mode the air pressure modulation means is disposed within the air flow path to provide the air pressure modulation, and in the second mode the air pressure modulation means is not disposed within the air flow path.

The proposed system provides a highly efficient and simple means of facilitating controllable pressure flow to the cuff, such that, in the context of hemodynamic monitoring, air pressure provided to the cuff can be varied for different patients and different use cases. An alternative approach would be to provide an air pump and associated driving electronics which is capable of electronically adapting the pressure of air supplied. However, this is a complex and relatively resource expensive solution and, for most cases, represents an overengineering of the solution. According to the proposed concept presented herein, different pressure modes are achieved in a manner that does not require any modification to the generator of the air source itself and can be implemented at any point along the downstream fluid path to the cuff. In some embodiments, it may be implemented inside the device which generates the air flow (e.g. a patient monitor), or in further embodiments it may be implemented in a separate external unit connectable in-line along the hosing to the cuff, the latter advantageously enabling the solution to be implemented in a retro-fit fashion to any existing blood pressure measurement apparatus. It may also be considered to integrate the air pressure modulation means within the cuff itself.

As will be explained later, one motivation behind the making of this invention was a need to adapt the air pressure in the context of a novel blood pressure measurement apparatus in development by the applicant which employs cuffs of different sizes for different patients (e.g. containing inflatable bladders of different volumes / volume capacities). In this context, due to the different size of the cuffs, the air pressure needs to be modified for use with each one. However, the provided invention can be applied generally for air flow modification in hemodynamic measurement systems in any context and for any use case.

There are different particular ways to implement the switching between the modes.

Two main categories are: active switching, wherein the system includes means for automatically or actively changing the mode; and manual switching, wherein the system includes means permitting a user or operator to selectively use the device in the two different modes.

In either case, the air pressure modulation means may be switchable in or out of the air flow path to either provide the air pressure modulation or not provide the air pressure modulation. An alternative approach would be to have the air pressure modulation means permanently connected into the air flow path, but wherein a level or degree of air pressure modulation is controllable.

In some embodiments, the system may include a switching mechanism to facilitate physical switching in or out of the air pressure modulation means to the air flow path. The physical switching may involve changing a configuration of a switchable valve between two outflow paths for example.

In some embodiments, the air flow path includes two or more selectable (e.g., fluidically parallel) air flow branches, wherein one of the branches includes the air pressure modulation means and at least one of the branches does not include the air pressure modulation means. Selectable means that the air flow path is configurable to selectively include or not include each of the air flow branches. Selection of the air flow branches for inclusion may be exclusive, so that only one can be connected at any one time. Alternatively, the system may permit inclusion of more than one of the branches in the air flow path. One or more branches may be connected in a partial state via a mixing junction, so that a mix of two branches can be included.

For example, in some embodiments, the system includes a controllable air flow junction which is switchable between two or more, e.g., fluidically parallel, air flow branches, wherein one of the branches includes the air pressure modulation means and at least one of the branches does not include the air pressure modulation means, e.g., a T-junction or Yjunction. This junction may be electronically controllable. As will be explained later, an alternative approach is to provide both of the two branches connected to an outlet of the air source in a static fashion, and wherein a user simply connects the tubing leading to the cuff to an outflow port of a selected one of the two branches, while the other of the two branches has an outflow port which remains sealed.

In some embodiments, the aforementioned air flow junction permits connection into the air flow path of only one of the branches at a time. Alternatively, the air flow junction may permit partial connection of multiple of the branches at a same time. This provides a means for greater configurability of the provided air pressure; by varying the degree to which the branch containing the modulation means is connected into the fluid path, the level of pressure modulation can be varied. Thus, this makes the configuration more flexible and enables more precise regulation of the air flow towards the cuff.

In some embodiments, the system includes a control module comprising a display for displaying one or more physiological parameters of the patient; and wherein the source of pressurized air is integrated in the control module. The control module may be a patient monitor unit for example.

In some embodiments, the air pressure modulation means is integrated in the control module, e.g., is integrated inside a housing of the unit.

In some embodiments, the control module includes: a first branch of the air flow path which connects through the air pressure modulation means and which terminates at a first air outflow port of the control module; and a second branch of the air flow path which does not connect through the air pressure modulation means and which terminates at a second air outflow port of the control module. In this way, the user is able to select between the first or second mode of operation simply by selectively connecting a tubing of the cuff to the first or the second outflow port.

The air pressure modulation means could be integrated inside the control module housing, or could be a unit attached to a side of the control module with its own outflow port.

The cuff may comprise a fluid inflow tubing connecting to the bladder, and wherein the fluid inflow tubing is adapted to fluidly connect with each of the first and second outflow port of the control module.

Instead of integrating the air pressure modulation means inside the control module, the air pressure modulation means may be incorporated in a separate unit to the control module.

In this case, in some embodiments, the control module may include a first fluid interface arrangement adapted to fluidically couple with a second fluid interface arrangement comprised by said separate unit, wherein, when so coupled, the air pressure modulation means is disposed within said air flow path, and wherein, when not so coupled, the air pressure modulation means is not disposed within the airflow path.

Thus, in this embodiment, the air pressure modulation means is simply an extra module of the patient monitor, and wherein a system permits a user to 'plug-in' the modulation means. The patient monitor may be configured to automatically detect the docking.

In some embodiments, a portion of the air flow path inside the control module may include a first branch which is connected to said first fluid interface arrangement such that, when the separate unit is fluidically coupled to the first fluid interface arrangement, the air pressure modulation means is connected along the air flow path, a second, by-pass branch, arranged such that, when the separate unit is not fluidically coupled to the first fluid interface arrangement, the air flow from the source of pressurized air passes through the by-pass branch. In other words, there may be a separate outflow port on the patient monitor for use in connecting to the tubing of the cuff when the pressure modulation means is not attached. This allows the fluid interface arrangement for connecting to the separate unit to be of a different type or shape to the connector needed to attach directly to the cuff tubing.

In some embodiments, the control module may include a docking arrangement, wherein the docking arrangement includes said first fluid interface arrangement and includes mechanical attachment means for mechanically coupling with the separate unit.

The separate unit may be structurally configured to dock with said docking arrangement and to be mechanically coupled with the mechanical attachment means to thereby hold the separate unit to the control module.

This effectively provides a 'click-dock' attachment means.

With regards to the pressure modulation means, there are different options for implementing this.

In an advantageously simple set of embodiments, the air pressure modulation means comprises simply a fluid chamber or reservoir for expanding a total volume available to receive air provided by the source of pressurized air. When connected into the fluid supply path, this reduces the flow pressure supplied to the cuff by an amount related to the fluid chamber volume.

Other alternatives are possible.

In some embodiments, the air pressure modulation means comprises a vent valve for venting a portion of the air provided by the source of pressurized air out of the air flow path, e.g. into the atmosphere.

In further embodiments, the air pressure modulation means may include a fluid resistor component, such as a hydrostatic resistor component. Here, a fluid resistor is used which, when connected in the fluid path, adds a hydrostatic resistance. This represents an additional pneumatic resistance to the pressurized air.

In some embodiments, the fluid resistor component comprises a reduced-width tube section for incorporation within said air flow path. For example, this may comprise a hose section of smaller diameter than the hose surrounding the section. For example, in some embodiments, the air flow path includes one or more air flow tubes, wherein the reduced width tube section has a smaller diameter than a minimum diameter of the one or more air flow tubes of the air flow path.

In some embodiments, the fluid resistor component comprises a variable fluid resistor.

In some embodiments, the variable fluid resistor may comprise: a tubing section comprising a flexible (e.g., elastic) tubing wall; and a pressure chamber having an adjustable internal pressure atmosphere, wherein the tubing section is at least partially located inside the pressure chamber. The variable fluid resistor may optionally further comprise a controller for controlling the internal pressure atmosphere of the pressure chamber for thereby controlling a variable compression of the elastic tubing wall to thereby adjust an internal diameter of the tubing section.

In some embodiments, the variable fluid resistor comprises: a tubing section comprising a flexible (e.g., elastic) tubing wall; and an electromechanical actuation means for adjusting a compression state of the flexible tubing wall for thereby varying an internal diameter of the tubing section.

In some embodiments, the system may comprise a first and second cuff, the second cuff having a smaller bladder (e.g. smaller bladder volume) than the first; and wherein the system is selectively operable using either the first or second cuff connected to the air flow path. For example, the second cuff may be designed for application to a child, and is designed for wrapping around a body part with smaller outer diameter.

In some embodiments, the cuff comprises a tissue pressure sensor arranged for sensing a pressure between a surface of the body part and the cuff.

In some embodiments, the cuff includes a shell portion, wherein the shell portion is arranged so as to be located between the bladder and the body part when the cuff is wrapped around the body part; wherein the shell portion is arranged to surround the body part when the cuff is wrapped around the body part; and wherein the shell portion comprises a plurality of overlapping sections configured to slide relatively to each other, permitting variation of the diameter of the shell portion responsive to application of pressure by the actuator.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates components of an example system in accordance with one or more embodiments of the invention;
Fig. 2 illustrates a switching mechanism for transitioning between a first and second mode in accordance with one or more embodiments;
Fig. 3 illustrates a further example configuration in which the pressure modulation means is integrated in a patient monitor device and with active switching control;
Fig. 4 illustrates a further example configuration in which the pressure modulation means is external to a patient monitor device and having active switching control;
Fig. 5 illustrates a further example configuration in which the pressure modulation means is external to a patient monitor device and having active switching control;
Fig. 6 illustrates a further example configuration in which the pressure modulation means is integrated in a patient monitor device and with manual switching control;
Fig. 7 illustrates a further example configuration in which the pressure modulation means is external to a patient monitor device and having manual switching control;
Fig. 8 illustrates a further example configuration in which the pressure modulation means is external to a patient monitor device and having manual switching control;
Fig. 9 illustrates a further example configuration in which the pressure modulation means is external to a patient monitor device and having manual switching control;
Fig. 10 illustrates a further example configuration in which the pressure modulation means is external to a patient monitor device and having manual switching control;
Fig. 11 illustrates an example pressure modulation means in accordance with one or more embodiments, employing use of an air vent;
Fig. 12 illustrates a further example pressure modulation means in accordance with one or more embodiments, employing use of a fluid resistor; and
Fig. 13 illustrates a further example pressure modulation means in accordance with one or more embodiments, employing use of an alternate type of fluid resistor.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a development in the field of non-invasive hemodynamic monitoring systems, wherein a pressure or flow modulator is incorporated in-line along at least a portion of the fluid supply path to a measurement cuff, and wherein the system includes static or active means for permitting a user to selectively operate the system in a first mode in which the pressure modulator is active and a second mode in which the pressure modulator is not active, and optionally a third mode in which the pressure modulator is partially active.

In one example, a switch valve is used permitting switching of the pressure modulator in or out of the air flow circuit. This might be referred to as an active mode selection means. In another example, two air supply branches are provided in an air supply module with independent respective outlet ports on a working face of the air supply module, wherein only one branch includes the pressure modulator, and whereby a user is permitted to selectively use the first branch or the second branch by connecting a tubing of the cuff to one outlet port or another. This might be referred to as a static or passive mode selection means.

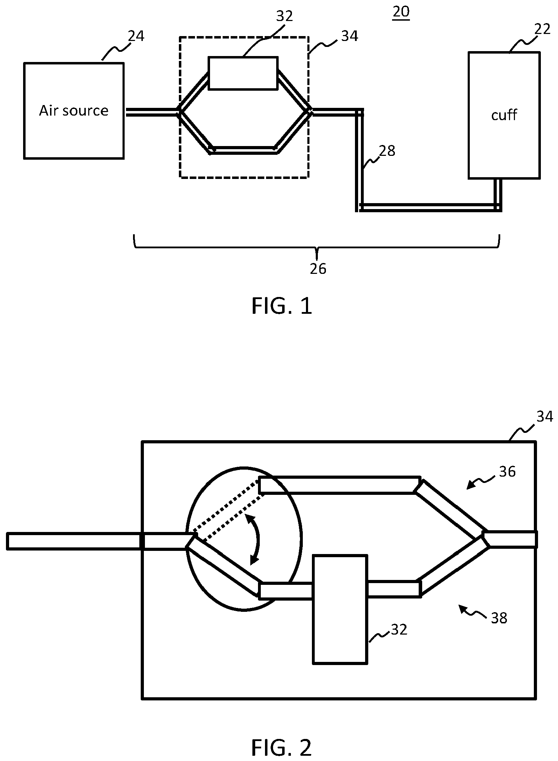

Fig. 1 illustrates components of an example system according to one or more embodiments. The system 20 comprises a cuff 22 with an inflatable bladder for adjusting a pressure applied by the cuff to a body part of a patient around which the cuff is worn.

The system 20 further comprises a source 24 of pressurized air for inflating the bladder of the cuff. For example, this comprises a pressurized air generator. For example, this comprises an air pump or an air condenser.

The air source 24 and the cuff 22 are connected by an air flow path 26. The air flow path may be facilitated by a plurality of different components and sections, which may include one or more conduits, tubes, hoses, chambers, reservoirs, valves and so on. For example, some portions of the air flow path may be integrated in a housing which houses the air source and some may be formed by the tubing 28 which connects to the cuff 22.

The system 20 further comprises an air pressure modulation means 32 for disposal along the air flow path 26 between the pressurized air source 24 and the cuff 22 and adapted to provide a modulation of a pressure of air arriving at the cuff.

The system is selectively configurable in each of at least two modes: a first mode in which the air pressure modulation means is arranged so as to provide the air pressure modulation, and a second mode in which the air pressure modulation means is arranged so as to not provide the air pressure modulation. The first mode may include various sub-modes in which different levels or degrees of air pressure modulation are applied. For example, the system may in some embodiments be configurable in a third mode which is a mix between the first and second modes, representing partial air pressure modulation. For example, instead of using a 2 state switch (or valves) a switch/valve with intermediate states could be used, controlled by the electronics/logic of a control module.

In some embodiments, in the first mode, the air pressure modulation means is disposed within the air flow path to provide the air pressure modulation, and in the second mode the air pressure modulation means is not disposed within the air flow path.

For example, in one set of embodiments, the selective operability in the first and second modes is facilitated by having the air pressure modulation means 32 be switchable in or out of the air flow path 26 to either provide the air pressure modulation or not provide the air pressure modulation. In alternative embodiments, the operability in the first and second modes is facilitated by modifying a physical state of the air pressure modulation means, e.g., opening or closing a vent.

In some embodiments, and as illustrated in Fig. 1 and Fig. 2, the switching may be facilitated by a switching mechanism 34 which is adapted to facilitate physical switching in or out of the air pressure modulation means to the air flow path. The switching mechanism may be an active switching mechanism. The switching mechanism may be a mechatronic switching mechanism. The switching mechanism may include electronics adapted to switch the pressure modulation means 32 in or out of the air flow path responsive to receipt of an electronic control signal, for example generated by a processor of a control module. In some embodiments, the air pressure modulation means and the air source 24 may be integrated in a control module of the system. Alternatively, the switching mechanism may be located outside of the control module.

With reference to Fig. 2, in some embodiments the switching mechanism 34 may comprise a controllable air flow junction which is switchable between two or more fluidically parallel air flow branches 36, 38, wherein one of the branches 38 includes the air pressure modulation means 32 and at least one of the branches 36 does not include the air pressure modulation means. For example, this may be a T-junction or Yjunction. To facilitate the switching, an electronic valve mechanism may be used, such as a solenoid valve.

In some embodiments, the system may further comprise a processor for performing control of the configuration of the switching mechanism 34. For example, a control line may be provided between the processor and the switching mechanism. The system 20 may include a control module in which the processor is integrated.

In some embodiments, the air flow junction permits connection into the air flow path of only one of the branches at a time. Thus, the switching mechanism either connects the upper branch 36 (for one mode) or the lower branch 38, including the pressure modulator 32, to the outlet of the air source 24. Instead of one single T-/Y-switch, two simple open/close switching devices (with always opposite respective states) could be used, one in the upper branch and one in the lower branch.

Alternatively, in order to make the configuration more flexible and to more precisely regulate the air flow towards the cuff, the air flow junction may permit partial connection of multiple of the branches at a same time. In other words, semi-states of the switch configuration can be used, i.e. both switches could be open to a certain fraction. This could be facilitated by a mixer valve which permits different fractions of the first 36 and second 38 branch to be connected into the air flow path, varying in graduated fashion between 100%:0% of branch 1:branch 2 and 0%: 100% of branch 1:branch 2.

There are different options as to how the air pressure modulation means 32 can be located within the system.

In some embodiments, the air pressure modulation means 32 may be integrated within a same unit which houses the pressurized air source 24. As noted previously, in some embodiments, the system includes a control module 42 and wherein the source of pressurized air 24 is integrated in the control module. The control module may comprise a display for displaying one or more physiological parameters of the patient. The control module may be a patient monitor unit in some examples.

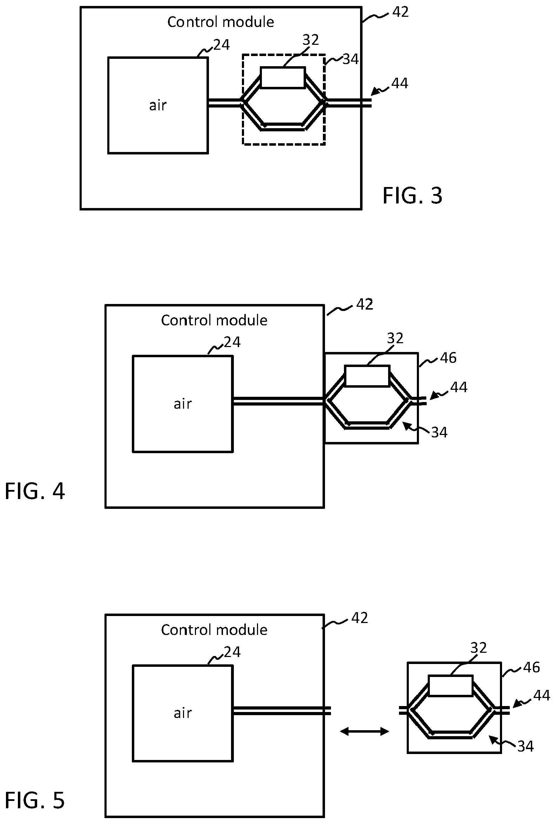

As illustrated in Fig. 3, in some embodiments, air pressure modulation means 32 may be integrated in the control module 42. In the example of Fig. 3, the control module 42 integrates the air pressure modulation means 32 and the switching mechanism 34 for controlling switching between the two modes. The control module includes an air outflow port 44 for connecting to a tubing 28 to supply air to the cuff 22. An outlet of the switching mechanism 34 is fluidically connected to the air outflow port 44 of the control module 42 so that whichever branch of the switching mechanism is active is connected to the air outflow port. In this way, configuration in either the first or second mode is facilitated by control of the switching mechanism 34.

An alternative to fully integrating the air pressure modulation means 32 inside in the control module is to provide a separate secondary unit which attaches to the control module 42, and wherein the secondary unit incorporates the air pressure modulation means 32.

Fig. 4 shows one example, in which a secondary unit 46 is provided, and wherein the secondary unit 46 includes the air pressure modulation means 32 and the switching mechanism 34 for switching the modulation means in and out of the air flow path. The secondary unit is attached fixedly or removably to an exterior of a housing of the control module 42, with an inflow port of the secondary unit connected fluidly with an air outflow port of the control module. For example, the control module includes a first fluid interface arrangement adapted to fluidically couple with a second fluid interface arrangement comprised by the secondary unit.

The secondary unit may be permanently attached to the control module 42, or may be releasably attached, as illustrated schematically in Fig. 5.

In either case, with the secondary unit 46 attached, effectively, the outflow port or terminal of the control module 42 is extended so that the user connects the tubing 28 of the cuff 22 to the outflow port 44 of the secondary unit 46. This arrangement has the advantage that it allows the invention to be retrofitted to existing hemodynamic measurement systems by attachment of the secondary unit to the control module of the existing system.

As an alternative to the active switching approach, the two selectable modes can be facilitated through a passive selection approach.

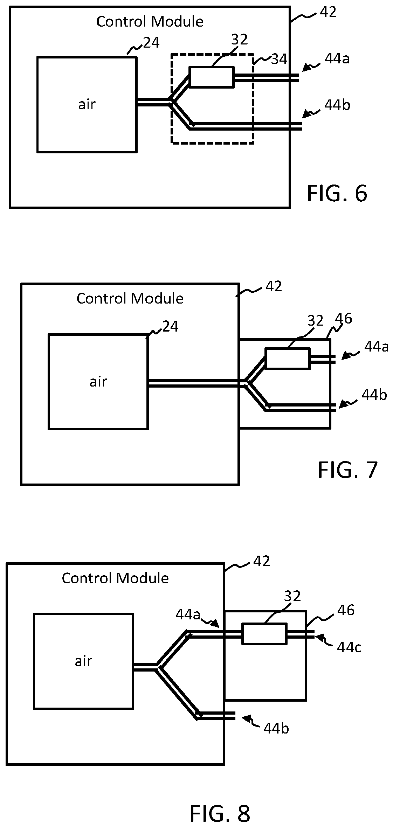

An example is illustrated in Fig. 6. Here, the air flow path includes a branching structure in which an outflow from the source of pressurized air 24 branches into two fluidically parallel paths, wherein the air pressure modulation means 32 is attached along one of the branches. Each of the two branches extends to a respective air outflow port 44a, 44b, and wherein each air outflow port is connectable to a tubing 28 of the cuff 22. The user is then able to select which of the two air outflow ports 44a, 44b to connect to the tubing of the cuff and thereby select which mode of operation the device should operate in. Each air outflow port may have a self-closing valve that closes the port when a tubing is not connected. In this way, the branch which is not connected to the tubing is kept sealed to prevent escape of gas.

In some embodiments, and as illustrated in Fig. 6, the branching structure and the air flow modulation means 32 can be integrated inside the control module 42, so that the control module 42 comprises the two air outflow ports 44a, 44b, for example at a working face or surface of the housing of the control module 42. In this example, the control module 42 includes a first branch of the air flow path which connects through the air pressure modulation means and which terminates at a first air outflow port 44a of the control module; and a second branch of the air flow path which does not connect through the air pressure modulation means and which terminates at a second air outflow port 44b of the control module. The cuff comprises a fluid inflow tubing connecting to the bladder, and wherein the fluid inflow tubing is adapted to be fluidly connectable with each of the first and second outflow port of the control module.

In other examples, and as illustrated in Fig. 7, the branching structure and the air pressure modulation means 32 may be integrated in a separate secondary unit 46 which attaches to the control module 42. Here, the secondary unit is attached to an exterior of a housing of the control module 42, with an inflow port of the secondary unit connected fluidly with an air outflow port of the control module. For example, the control module includes a first fluid interface arrangement adapted to fluidically couple with a second fluid interface arrangement comprised by the secondary unit. The secondary unit may be permanently attached to the control module 42, or may be releasably attached in some embodiments.

Another option is illustrated in Fig. 8. Here, the branching structure in the air flow path is provided inside the control module 42 but the air pressure modulation means 32 is provided integrated in a secondary unit 46 which attaches to the control module 42. The control module has a first 44a air outflow port which leads from the first branch of the air flow path and a second air outflow port 44b which leads from the second branch of the air flow path. The secondary unit 46 attaches to the control module such that an air inflow port of the secondary unit 46 fluidically couples with the first 44a air outflow port of the control module. The secondary unit incorporates the air pressure modulation means 32 and includes an air outflow port 44c leading from the air pressure modulation means. As illustrated in Fig. 8, this allows a user to selectively operate the system in the first mode or the second mode by connecting the tubing of the cuff to either the remaining free air outflow port 44b of the control module or to the air outflow port 44c of the secondary module 46.

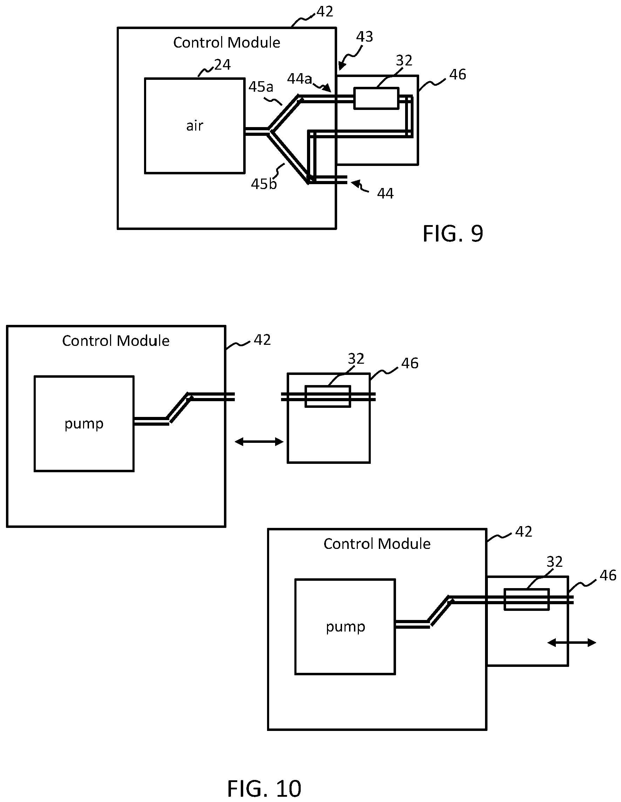

Another option is illustrated in Fig. 9. Here, the air pressure modulation means 32 is incorporated in a separate secondary unit 46. The control module 42 includes a first fluid interface arrangement 43 adapted to fluidically couple with a second fluid interface arrangement comprised by said separate unit, wherein, when so coupled, the air pressure modulation means 32 is disposed within the air flow path, and wherein, when not so coupled, the air pressure modulation means is not disposed within the airflow path. Fig. 9 shows the secondary unit 46 coupled to the fluid interface 43. The control module 42 further comprises a fluid outflow port 44 for coupling to tubing 28 which extends to the cuff. When the secondary module 46 is attached to the fluid interface 43 of the control module 42, the fluid from the air source 24 flows through a first air flow branch 45a in the control module 42, into the secondary unit 46, through the air pressure modulation means 32 in the secondary unit and back into the patient monitor for outflow through the outflow port 44. Thus, in this embodiment, the air pressure modulation means is effectively formed by an additional module 46 of the patient monitor which can be `plugged in' to the control module. It may be automatically detected.

In the illustrated example of Fig. 9, in addition to the first branch 45a, which is connected to said first fluid interface arrangement 43, a second, by-pass branch 45b is arranged such that, when the secondary unit 46 is not fluidically coupled to the first fluid interface arrangement, the air flow from the source 24 of pressurized air passes through the by-pass branch and out of the air outflow port 44. Thus the device can be used in the first mode or second mode by either attaching the secondary unit to the patient monitor or detaching the secondary unit from the patient monitor.

In accordance with any of the above described examples in which the secondary unit is attachable to the control module, the control module may include a docking arrangement, wherein the docking arrangement includes a first fluid interface arrangement and includes mechanical attachment means for mechanically coupling with the secondary unit 46, wherein the separate unit is structurally configured to dock with said docking arrangement and to be mechanically coupled with the mechanical attachment means to thereby hold the separate unit to the control module. For example, a click-dock attachment means is provided.

Another option is shown in Fig. 10. Here, the air pressure modulation means 32 is provided integrated into a secondary unit 46 which is separate to the control module 42. The secondary unit 46 is removably coupleable to the control module. Upon coupling an inflow portion of the secondary unit 46 it fluidically couples to an air outflow port of the control module. The switching between the first and second modes may be achieved by a user removing the secondary unit 46 or attaching the secondary unit. In the detached state, the user may connect the tubing of the cuff 22 to the air outflow port of the control module, in which case no air pressure modulation is applied. In the attached state, the user may connect the tubing of the cuff 22 to the air outflow port of the secondary unit 46 such that air pressure modulation is provided by the air pressure modulation means 32 integrated in the secondary unit 42.

With regards to the air pressure modulation means 32, different implementation options are possible.

According to a simple first example, the air pressure modulation means may comprise a fluid chamber or reservoir for expanding a total volume available to receive air provided by the source of pressurized air. The fluid chamber may be formed for example by a cavity delimited in the control module 42 or a secondary unit 46. By expanding the total volume of the air flow path, the pressure supplied to the cuff bladder is reduced during at least a beginning portion of an inflation cycle. As will be explained later, this is of particular value in the context of a system in which different sizes of cuff may be used in combination with different of the modes of the system.

With regards to the structure of the fluid chamber, the chamber may be defined by an internal cavity of a unit of air-tight material, e.g. plastic or metal, with an air inlet and an air outlet. The fluid chamber may be connected along the fluid supply path between the source of pressurized air and the cuff. The size of the volume defined by the fluid chamber may be adjustable. In some embodiments, the size of the volume defined by the fluid chamber may be adjustable manually by a user. For example one or more walls of the fluid chamber may be moveable along a path to vary a volume defined by the chamber and wherein this movement is actuable by a manual control. Alternatively, the volume defined by the fluid chamber may be adjustable electronically, for example with an electronic actuation means for effecting said movement of a wall of the chamber. According to some embodiments, the fluid chamber may be provided by a flexible bladder instead of a rigid vessel, the flexible bladder for example defining either a linear or non-linear expansion function.

By way of further example, an air pressure modulation means may in some embodiments comprise a vent valve for venting a portion of the air provided by the source of pressurized air out of the air flow path, e.g. into the atmosphere.

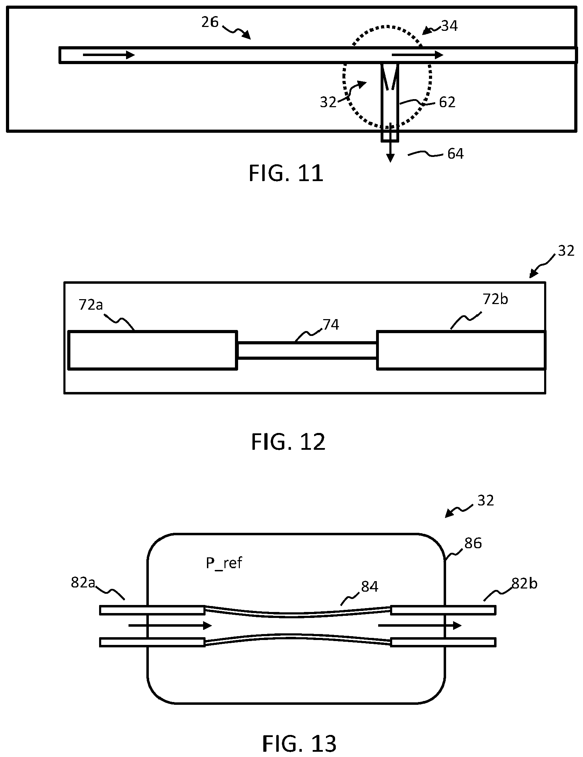

Fig. 11 shows one example of an air outflow vent 62. A switching mechanism 34 is provided which permits switching of the atmospheric vent in or out of the air flow path 26, i.e., opening or closing the vent to the pressurized air in the air flow path.

For example, the switching mechanism 34 may employ use of an electronic valve which is controllable to open or close an outflow to the atmosphere 64. By opening a vent to the atmosphere, a pressure of the pressurized air in the air flow path would be reduced, leading to a slower inflation of the cuff. In the first mode, the vent would be in an open state such that reduction of the air pressure is achieved. In the second mode, the vent would be closed to the air flow path so that no pressure modulation is applied. Thus connecting the air pressure modulation means in and out of the air flow path in this example comprises opening or closing the vent to the air flow path. In some examples, the air pressure modulation means 32 may comprise a permanently open vent, and wherein the previously discussed switching mechanism 34 is used to switch either in or out of the air flow path an air flow branch along which the vent is disposed. In further examples, there is instead provided a vent with a controllable open/close state, this being controllable by an electronic valve, and wherein this electronic valve forms the switching mechanism to permit switching the vent in or out of the air flow path, and thus to open or closed state.

In some examples, the switching means may permit a plurality of different open/close states representing different partial open/close states. Each of the plurality of vent open/close states may represent in practice a different total air outflow area provided by the vent. Thus, here, the system is selectively configurable in at least three modes, one mode in which the vent to the atmosphere is not fluidically coupled to the pressurized air in the air flow path (e.g., the vent is switched to a closed state) and then at least two modes, in each of which the vent to the atmosphere is fluidically coupled to the pressurized air in the air flow path (e.g., the vent is switched to an open state) and provides a different total air outflow area. This allows for greater control over the inflation cycle and may permit the system to be used with any of a wide variety of cuffs.

It is noted that in some embodiments, the air pressure modulation means may comprise both an air reservoir/chamber and a vent valve in combination.

By way of further example, in some embodiments, the air pressure modulation means includes a fluid resistor. The fluid resistor may for example be a hydrostatic resistor component. A hydrostatic resistor is a component that induces or exerts a hydrostatic resistance.

One simple example is illustrated in Fig. 12. Here, the hydrostatic resistor component comprises simply a reduced-width tube section 74 for incorporation within or along said air flow path. For example, and as illustrated in Fig. 12, a hydrostatic resistance can be achieved by means of adding a smaller width tube section part way along the air flow path. The reduced width tube section means a tube section for example of smaller diameter than surrounding sections 72a, 72b of tubing. For example, the air flow path includes one or more air flow tubes, wherein the reduced width tube section has a smaller diameter than a minimum diameter of the one or more air flow tubes of the air flow path.

The hydrostatic resistor adds an additional pneumatic resistance to the pressurized air. Since the source of pressurized air (e.g., the air pump) is limited in air flow and pressure, the flow rate of air able to travel through the hydrostatic resistance will be reduced, which will result in a slower inflation of a connected cuff.

In some embodiments, the pressure modulation means 32 may be integrated in a portion of the cuff or a portion of the tubing leading to the cuff, for example with a switching mechanism to facilitate switching the pressure modulation means in or out of the air flow path. In some embodiments, and as will be described later, there may be two cuffs, and wherein the pressure modulation means 32 is integrated along the fluid supply line to only one of the cuffs, and wherein a switching mechanism is adapted to permit switching of the supply of pressurized air between the fluid supply line for one cuff and the fluid supply line for the other cuff.

In accordance with a further one or more embodiments, a pressure modulation means 32 in the form of a fluid resistor may be provided and wherein the fluid resistor has a variable resistance. The fluid resistor may be a hydrodynamic fluid resistor component.

An example is illustrated schematically in Fig. 13. Here, the pressure modulation means 32 comprises a fluid resistor which comprises a flexible tubing section 84 incorporated along the fluid supply path, for example with non-flexible (or less flexible) tubing sections 82a, 82b extending to and from the flexible section. The flexible tubing section is located at least partially inside a pressure chamber 86 having a reference pressure, P_ref. The internal pressure atmosphere, P_ref, of the pressure chamber may be configured as desired so as to induce a deformation of the flexible tubing wall 84 which results in a desired level of fluid resistance. As illustrated in Fig. 12, if the internal pressure atmosphere, P_ref, is configured at a higher pressure than the pressurized air supplied along the fluid supply path, then a compression of the flexible tubing wall 84 results, with the effect of creating a narrowed width tubing section. Thus reduces the airflow, and thus reduces the pressure of air delivered to the cuff.

The pressure modulator may be configured in some embodiments such that the internal pressure atmosphere P_ref is fixed at a pre-defined level in manufacture, according to application requirements. According to a further set of examples, the pressure chamber may have an adjustable internal pressure atmosphere, and wherein a controller is provided for controlling the internal pressure atmosphere, P_ref, of the pressure chamber for controlling a variable compression of the elastic tubing wall to thereby adjust an internal diameter of the tubing section. This results in a fluid resistor with an adjustable fluid resistance.

An alternative means for implementing a variable hydrodynamic resistor is to provide the tubing section 84 comprising a flexible (e.g. elastic) tubing wall, and to further provide an electromechanical actuation means for adjusting a compression state of the flexible tubing wall for thereby varying an internal diameter of the tubing section. Here, an electromechanical actuator is provided which engages with the tubing to apply a controllable compression. The electromechanical actuation means can replace the pressure chamber 86.

As already discussed, the general inventive concept admits of a wide variety of possible application. In general, the proposed system provides a mechanically and functionally simple way of enabling modification or customization of the pneumatic properties of a delivered source of pressurized air, in particular varying a pressure level of the air. This is achieved advantageously without the need for a dynamically controllable pump, and thus can be implemented even by way of retrofit of existing hemodynamic monitoring systems which use an inflatable cuff.

There will now be discussed one particular use case for which the inventors have found the proposed system particularly advantageous.

By way of background, an improved device developed by the inventors associated with the present application makes use of a novel cuff design, the use of which enables continuous monitoring. One problem encountered however is that the cuff design requires different sized cuffs to be used for patients with different arm sizes, most notably adults versus children.

In more detail, the improved cuff design comprises the following components. The cuff comprises an inflatable bladder, which may otherwise be referred to as the "actuator". This is supplied by an air hose or tubing. When inflated, the actuator compresses the cuff around the patient's upper arm (or any other limb).

A relatively rigid shell portion, referred to herein as the "shell", is surrounded and bound by the actuator. When the bladder is inflated, the shell portion serves to compress the patient's arm evenly and smoothly. The interior of the shell, when assembled, may be covered by a liner to prevent pinching of the patient. A sensor assembly is further included which comprises a fluid-filled pouch, referred herein as the sensor pad (SP), located so as to be between the shell portion and the limb during use of the cuff, i.e., on the interior side of the shell. This is connected by a fluid-filled tubing to a pressure transducer ("PT"). This may be disposable. It may be mountable on the exterior of the cuff. It may be connected by an electrical cable to the control module, e.g. to a patient monitor. When the actuator is inflated, the shell presses the sensor pad against the patient's inner upper arm, and the tissue pressure of the patient's arm is hydraulically conducted to the PT. The PT transduces the fluid pressure to an analog electrical signal which is conducted by means of the electrical cable to the control module, where the brachial arterial pulsation (beat-to-beat blood flow) is displayed and analyzed.

According to at least one set of examples, this cuff is a non-sterile, single-patient-use device, intended to be used on a single patient for no more than three days (72 hours). According to at least one set of examples, the cuff is intended for use on adult critical care patients in the operating room (OR) or intensive care unit (ICU) who are intubated and require fluid management.

The cuff enables non-invasive monitoring of blood pressure and a variety of other different hemodynamic parameters.

In use, the cuff is placed on the patient's upper arm and is connected to a control module, e.g., a patient monitor, by means of an electrical cable and an air hose or tubing. With regards to the electrical cable, optionally, the pressure transducer may contain an authentication circuitry (e.g. a 1-wire authentication circuitry) to identify the cuff model and other information stored therein.

According to at least one set of examples, the cuff is available in four different cuff sizes to accommodate different arm sizes. These include: small adult (SA), adult (A), large adult (LA), and large adult short (LA-S). For each of these, the volume size of the bladder is different. Some of the cuffs differ in respect of the diameter of arm that they are designed to fit to.

In order to obtain accurate measurement, a relatively slow airflow is required at a beginning of the inflation cycle. For smaller cuff sizes (e.g., Small Adult), the flow rate provided by the pump during the beginning of the inflation cycle is too high, causing the cuff to inflate too quickly, due to the smaller bladder volume compared to the larger cuffs.

To compensate for this, the inventors have recognized that there would be value in providing a pressure modulator in line along the airflow path to modify the pressure characteristics when the system is being used with a smaller cuff size. However, a potential weakness identified by the inventors with this solution is poor usability due to the need to manually connect the pressure modulator when using the smaller cuff. This additional step in the workflow is an additional burden for the operator. Another disadvantage is the fact that it may cause some risk of malfunction if not connected correctly (e.g., without a fully fluid tight connection with the tubing).

Thus, the inventors have recognized that a more advantageous solution would be to provide a system which is selectively operable in each of two modes, at least a first mode in which a pressure modulator is arranged so as to provide the air pressure modulation, and a second mode in which the air pressure modulation means is arranged so as to not provide the air pressure modulation.

With this use case in mind, according to one set of embodiments, the system may include at least a first cuff and second cuff. The second cuff may have a smaller (volume) bladder than the first and/or may permit attachment to a limb of smaller maximum diameter than the first. The system in this set of embodiments is selectively operable using either the first or second cuff connected to the air flow path. The second cuff may be designed for application to a child or small adult for example, and is designed for wrapping around a body part with smaller outer diameter.

In some embodiments, the first mode is used when the first cuff is connected to the air flow path and the second mode is used when the second cuff is connected to the air flow path.

In some embodiments, the first or each cuff may comprise a tissue pressure sensor arranged for sensing a pressure between a surface of the body part and the cuff.

In some embodiments, the first or each cuff may include a shell portion wherein the shell portion is arranged so as to be located between the bladder and the body part when the cuff is wrapped around the body part. The shell portion may be arranged to surround the body part when the cuff is wrapped around the body part. The shell portion may comprise a plurality of overlapping sections configured to slide relatively to each other, permitting variation of the diameter of the shell portion responsive to application of pressure by the actuator.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (20) comprising:
a cuff (22) with an inflatable bladder for adjusting a pressure applied by the cuff to a body part of a patient around which the cuff is worn;
a source (24) of pressurized air for inflating the bladder of the cuff;
an air flow path (26) for fluidly connecting the source of pressurized air and the inflatable bladder of the cuff; and
an air pressure modulation means (32) for disposal along the air flow path between the source of pressurized air and the inflatable bladder of the cuff and adapted to provide a modulation of a pressure of air arriving at the inflatable bladder of the cuff,
wherein the system is selectively configurable in each of at least two modes: a first mode in which the air pressure modulation means is arranged so as to provide the air pressure modulation, and a second mode in which the air pressure modulation means is arranged so as to not provide the air pressure modulation.

2. The system of claim 1, wherein the air pressure modulation means is switchable in or out of the air flow path to either provide the air pressure modulation or not provide the air pressure modulation, and
optionally wherein the system includes a switching mechanism to facilitate physical switching in or out of the air pressure modulation means to the air flow path.

3. The system of claim 1 or 2, wherein the air flow path includes two or more selectable parallel air flow branches, wherein one of the branches includes the air pressure modulation means and at least one of the branches does not include the air pressure modulation means.

4. The system of any of claims 1-3, wherein the system includes a controllable air flow junction which is switchable between two or more parallel air flow branches, wherein one of the branches includes the air pressure modulation means and at least one of the branches does not include the air pressure modulation means, and
optionally wherein
the air flow junction permits connection into the air flow path of only one of the branches at a time; or
the air flow junction permits partial connection of multiple of the branches at a same time.

5. The system of any of claims 1-4,
wherein the air pressure modulation means comprises a fluid chamber or reservoir for expanding a total volume available to receive air provided by the source of pressurized air; or
wherein the air pressure modulation means comprises a vent valve for venting a portion of the air provided by the source of pressurized air out of the air flow path, e.g. into the atmosphere.

6. The system of any of claims 1-5, wherein the system includes a control module comprising a display for displaying one or more physiological parameters of the patient, and wherein the source of pressurized air is integrated in the control module, and optionally wherein the air pressure modulation means is integrated in the control module.

7. The system of claim 6, wherein the control module includes:
a first branch of the air flow path which connects through the air pressure modulation means and which terminates at a first air outflow port of the control module; and
a second branch of the air flow path which does not connect through the air pressure modulation means and which terminates at a second air outflow port of the control module; and
optionally wherein the cuff comprises a fluid inflow tubing connecting to the bladder, and wherein the fluid inflow tubing is adapted to be fluidly connectable with each of the first and second outflow port of the control module.

8. The system of any of claims 1-7,
wherein the air pressure modulation means is incorporated in a separate unit to the control module;
wherein the control module includes a first fluid interface arrangement adapted to fluidically couple with a second fluid interface arrangement comprised by said separate unit, wherein, when so coupled, the air pressure modulation means is disposed within said air flow path, and wherein, when not so coupled, the air pressure modulation means is not disposed within the airflow path.

9. The system of claim 8 wherein a portion of the air flow path inside the control module includes a first branch which is connected to said first fluid interface arrangement such that, when the separate unit is fluidically coupled to the first fluid interface arrangement, the air pressure modulation means is connected along the air flow path, a second, by-pass branch, arranged such that, when the separate unit is not fluidically coupled to the first fluid interface arrangement, the air flow from the source of pressurized air passes through the by-pass branch.

10. The system of any of claims 1-9, wherein the air pressure modulation means includes a fluid resistor component.

11. The system claim 10, wherein the fluid resistor component comprises a reduced-width tube section for incorporation within said air flow path, and optionally wherein the air flow path includes one or more air flow tubes, wherein the reduced width tube section has a smaller diameter than a minimum diameter of the one or more air flow tubes of the air flow path.

12. The system of claim 10-11, wherein the fluid resistor component comprises a variable fluid resistor.

13. The system of claim 12, wherein the variable fluid resistor comprises:
a tubing section comprising a flexible tubing wall;
a pressure chamber having an adjustable internal pressure atmosphere, wherein the tubing section is at least partially located inside the pressure chamber; and
optionally a controller for controlling the internal pressure atmosphere of the pressure chamber for thereby controlling a variable compression of the elastic tubing wall to thereby adjust an internal diameter of the tubing section.

14. The system of claim 12, wherein the variable fluid resistor comprises:
a tubing section comprising a flexible tubing wall; and
an electromechanical actuation means for adjusting a compression state of the flexible tubing wall for thereby varying an internal diameter of the tubing section.

15. The system of any of claims 1-14,
wherein the system includes a first and second cuff, the second cuff having a smaller bladder than the first; and
wherein the system is selectively operable using either the first or second cuff connected to the air flow path.
